Europäisches Patentamt

European Patent Office

Office européen des brevets

(19)

(11) Veröffentlichungsnummer: **0 100 940**
**B1**

(12) **EUROPÄISCHE PATENTSCHRIFT**

(45) Veröffentlichungstag der Patentschrift:
02.05.85

(51) Int. Cl.⁴: **C 07 C 125/067**

(21) Anmeldenummer: 83107154.3

(22) Anmeldetag: 21.07.83

(54) **Verfahren zur Herstellung von Brenzcatechinmethylcarbamat.**

(30) Priorität: 27.07.82 DE 3227931

(43) Veröffentlichungstag der Anmeldung:
22.02.84 Patentblatt 84/8

(45) Bekanntmachung des Hinweises auf die Patenterteilung:
02.05.85 Patentblatt 85/18

(84) Benannte Vertragsstaaten:
BE CH DE FR GB IT LI NL

(56) Entgegenhaltungen:
DE - A - 2 650 828

(73) Patentinhaber: BASF Aktiengesellschaft,
Carl-Bosch-Strasse 38, D-6700 Ludwigshafen (DE)

(72) Erfinder: Reissenweber, Gernot, Dr., Drosselstrasse 15,
D-6737 Boehl-Iggelheim (DE)
Erfinder: Kersten, Siegfried, Dr.,
Max-Slevogt-Strasse 21, D-6710 Frankenthal (DE)
Erfinder: Ditter, Walter, Feuerbachstrasse 7,
D-6900 Heidelberg (DE)
Erfinder: Jacobs, Peter, Dr., Kalmitstrasse 2,
D-6718 Gruenstadt (DE)

## Beschreibung

Die Erfindung betrifft ein Verfahren zur Herstellung von Brenzcatechinmonomethylcarbamat — einem Vorprodukt für gewisse Pflanzenschutzmittel — durch Umsetzung von Brenzcatechincarbonat mit Methylamin in Gegenwart eines tertiären Amins und vorzugsweise eines Lösungsmittels.

Verfahren zur Herstellung von Brenzcatechincarbamaten durch Umsetzung von Brenzcatechincarbonat mit primären oder sekundären Aminen sind beispielsweise aus Liebigs „Ann. Chem.", 300, 135 (1898); ibid., 562, 205 (1949), „Journal f. prakt. Chemie", 313, 626 (1971) sowie DE-OS Nr. 2650828 bekannt, vgl. auch Houben-Weyl, 4. Aufl., Bd. 8, S. 139/140.

Brenzcatechinmonomethylcarbonat wird nach der DE-A Nr. 2650828 in Gegenwart einer katalytischen Menge des Hydrochlorids eines quasiaromatischen Amins (z. B. des 2-Chlorpyridins) aus dem Carbonat erhalten, wobei das besagte Hydrochlorid dadurch in das Reaktionsgemisch gelangt, dass das Vorprodukt, eben Brenzcatechincarbonat, aus Brenzcatechin und Phosgen in Gegenwart einer geringen Menge an Amin hergestellt wird. Der freigesetzte Chlorwasserstoff (und überschüssiges Phosgen) kann natürlich nur in dem Masse entfernt werden, als er nicht vom Amin gebunden wird. Dieses Aminhydrochlorid ist nach den Angaben bei der folgenden Umsetzung des Carbamats mit Methylamin zugegen.

Die bekannten Verfahren haben — namentlich für das Herstellen im technisch wirtschaftlichen Massstab — eine Reihe von Nachteilen: Die relativ schwerlöslichen Carbamate scheiden sich aus den meisten gebräuchlichen Lösungsmitteln schon während der Umsetzung ab. Die entstehenden Suspensionen sind nur schwer zu fördern, was die kontinuierliche Herstellung erschwert. Besonders erschwert ist auch die Durchmischung der Reaktionspartner und die Abfuhr der Reaktionswärme, was zur Bildung von Nebenprodukten wie Brenzcatechin und N,N'-Dimethylharnstoff führt. Zwar existiert ein Lösungsmittel mit besseren Lösungseigenschaften, nämlich Aceton; beim Arbeiten in Aceton wird aber reichlich Brenzcatechin als Nebenprodukt gebildet, da aus Methylamin und Aceton in einem vorgelagerten Gleichgewicht die Schiffbase gebildet wird, und das dabei freiwerdende Wasser das Carbamat verseift. Ein anderes geeignetes Lösungsmittel, nämlich 1,4-Dioxan kann aus toxikologischen Gründen nicht verwendet werden.

Die Aufgabe, ein glatt verlaufendes Verfahren zur Herstellung von Brenzcatechinmonomethylcarbamat wird erfindungsgemäss gelöst, wenn man Brenzcatechincarbonat mit Methylamin in Gegenwart einer vorzugsweise stöchiometrischen Menge eines freien tertiären Amins umsetzt. Dabei hat der Zusatz des tertiären Amins zwei überraschende Folgen: Zum einen wird die Löslichkeit der Carbamate erheblich gesteigert, so dass beispielsweise in Methylenchlorid bis zu 30%ige Carbamatlösungen möglich sind und zum anderen verläuft die Umsetzung wesentlich schneller als ohne den Zusatz, erfindungsgemäss etwa um das vierfache, so dass eine entsprechend bessere Raum-Zeit-Ausbeute möglich ist.

Die Umsetzung verläuft bei einer Temperatur von −50 bis +50, vorzugsweise −20 bis +20° C mit ausreichender Geschwindigkeit.

Als Lösungsmittel werden chlorierte Kohlenwasserstoffe wie Dichlormethan, Dichlorethan, Dichlorethylen oder sogenannte aprotisch-polare Lösungsmittel eingesetzt. Solche aprotischpolaren Lösungsmittel, wie Tetrahydrofuran, Dimethylformamid oder -sulfoxid sind jedoch im allgemeinen kostspieliger.

Als tertiäre Amine lassen sich alle Amine der allgemeinen Formel $N(R)_3$ verwenden, wobei R einen verzweigten oder unverzweigten aliphatischen oder cycloaliphatischen Rest von beispielsweise bis zu 15 C-Atomen oder zu jeweils zwei gemeinsamen Ringgliedern eines cyclischen Amins bedeutet. Die Reste R können gleich oder verschieden sein. Als beispielhaft seien genannt: Triethylamin, Tri-n-propylamin, Tributylamin, Triisobutylamin, Tri-2-ethylhexylamin, N,N-Dimethylethylamin, N,N-Dimethylisopropylamin, N,N-Dimethyl-2-ethylhexylamin, N-Methylditridecylamin, Dimethylcyclohexylamin, N-Methyldicyclohexylamin und N-Methylhexamethylenimin. Quasiaromatische Amine wie Pyridin sind ebenfalls geeignet. Die zugesetzte Menge an tertiärem Amin beträgt 0,2 bis 1,4 mol, bezogen auf 1 mol Carbonat.

Zweckmässig legt man Brenzcatechincarbonat zusammen mit dem gewählten tertiären Amin in dem gewählten Lösungsmittel vor und führt unter kräftiger Durchmischung die stöchiometrische Menge Methylamin allmählich zu. Vorteilhaft bei technischer Ausführung kann man eine Lösung von Brenzcatechincarbonat und tertiärem Amin in einer Umlaufapparatur oder einem Reaktionsrohr kontinuierlich mit Methylamin zusammen zur Reaktion bringen. Brenzcatechincarbonat und tertiäres Amin können auch getrennt in Lösung zugeführt und mit Methylamin zusammen zur Reaktion gebracht werden.

Aus dem Reaktionsgemisch lässt sich das Carbamat gewinnen, indem man das tertiäre Amin in ein Salz überführt und dann das auskristallisierende Carbamat isoliert. Ggf. wird das Carbamat durch Waschen mit Wasser von Ammoniumsalzen befreit. Die Carbamatlösungen selbst können, wo die Gegenwart des Amins nicht stört, sofort für weitere Reaktionsschritte eingesetzt werden wie beispielsweise für Alkylierungen der freien Hydroxylgruppe.

Nach dem erfindungsgemässen Verfahren lässt sich Brenzcatechinmonomethylcarbamat in hoher Ausbeute und Reinheit darstellen. Gerade der Herstellung sehr reiner Produkte kommt in diesem Fall besondere Bedeutung zu, da die nach dem Verfahren der Erfindung hergestellten Carbamate wertvolle Zwischenprodukte für insektizide Wirkstoffe darstellen (DE-AS Nr. 2231249).

*Beispiel 1:*

Einer Lösung von 680 g Brenzcatechincarbonat und 555 g Triethylamin in 2,5 l Methylenchlorid wurden bei 5° C unter kräftigem Rühren 155 g gasförmiges Methylamin zugeführt. Dann wurde mit 500 g 36%iger Salzsäure versetzt, abgesaugt und mit Wasser gewaschen. Es wurden 801 g (96% der erwarteten Menge) Brenzcatechinmonomethylcarbamat vom Schmelzpunkt 125° C erhalten.

*Beispiel 2:*

Einer Umlaufapparatur mit einem Fassungsvermögen von 180 ml wurde eine Lösung aus jeweils 4 l Methylenchlorid, 1088 g Brenzcatechincarbonat und 1016 g N,N-Dimethylcyclohexylamin mit einer Zulaufgeschwindigkeit von 4,8 l/h und gleichzeitig über einen zweiten Zulauf 198 g Methylamin pro Stunde zugeführt. Die Temperatur wurde bei 10 bis 15° C gehalten. Die abfliessende Lösung wurde alle 20 min mittels HPLC analysiert; es erwies sich, dass der Umsatz von Brenzcatechincarbonat vollständig und das erhaltene Brenzcatechincarbamat von 96 bis 98%iger Reinheit war. Insgesamt 10 l einer so hergestellten Lösung wurden wie vorstehend beschrieben aufgearbeitet und ergaben 2080 g (93%) Brenzcatechinmonomethylcarbamat; Gehalt nach HPLC 99%.

## Patentansprüche

1. Verfahren zur Herstellung von Brenzcatechinmonomethylcarbamat durch Umsetzung von Brenzcatechincarbonat mit Methylamin, dadurch gekennzeichnet, dass man die Umsetzung in Gegenwart von 0,2 bis 1,4 mol, bezogen auf 1 mol Carbonat, eines freien tertiären Amins durchführt.

2. Verfahren nach Anspruch 1, dadurch gekennzeichnet, dass man die Umsetzung in einem halogenierten aliphatischen Kohlenwasserstoff oder einem aprotisch-polaren Lösungsmittel durchführt.

3. Verfahren nach Anspruch 2, dadurch gekennzeichnet, dass man die Umsetzung in Methylenchlorid, Tetrahydrofuran, Dimethylformamid oder Dimethylsulfoxid durchführt.

4. Verfahren nach einem der Ansprüche 1 bis 3, dadurch gekennzeichnet, dass man einen Überschuss an Methylamin vermeidet.

## Claims

1. A process for the preparation of pyrocatechol methylcarbamate by reacting pyrocatechol carbonate with methylamine, wherein the reaction is carried out in the presence of from 0.2 to 1.4 mol of a free tertiary amine per mole of carbonate.

2. A process as claimed in Claim 1, wherein the reaction is carried out in an aliphatic halohydrocarbon or an aprotic-polar solvent.

3. A process as claimed in Claim 2, wherein the reaction is carried out in methylene chloride, tetrahydrofuran, dimethylformamide or dimethylsulfoxide.

4. A process as claimed in any of Claims 1 to 3, wherein an excess of methylamine is avoided.

## Revendications

1. Procédé de préparation du monométhylcarbamate de pyrocatéchine par réaction du carbonate de pyrocatéchine avec la méthylamine, caractérisé en ce que la réaction est réalisée en présence de 0,2 à 1,4 mol d'une amine tertiaire libre par mole de carbonate.

2. Procédé suivant la revendication 1, caractérisé en ce que la réaction est effectuée dans un hydrocarbure aliphatique halogéné ou un solvant polaire aprotique.

3. Procédé suivant la revendication 2, caractérisé en ce que la réaction est effectuée dans du chlorure de méthylène, du tétrahydrofuranne, du diméthylformamide ou du diméthylsulfoxyde.

4. Procédé suivant l'une des revendications 1 à 3, caractérisé en ce que l'on évite la présence de la méthylamine en excès.